Europäisches Patentamt

European Patent Office

Office européen des brevets

(11) Veröffentlichungsnummer: **0 259 921**
**A2**

(12) **EUROPÄISCHE PATENTANMELDUNG**

(21) Anmeldenummer: 87201638.1

(51) Int. Cl.⁴: **G01N 23/20**

(22) Anmeldetag: 31.08.87

(30) Priorität: 09.09.86 DE 3630651

(43) Veröffentlichungstag der Anmeldung:
16.03.88 Patentblatt 88/11

(84) Benannte Vertragsstaaten:
**DE FR GB NL**

(71) Anmelder: **Philips Patentverwaltung GmbH**
**Wendenstrasse 35 Postfach 10 51 49**
**D-2000 Hamburg 1(DE)**

(84) **DE**

(71) Anmelder: **N.V. Philips' Gloeilampenfabrieken**
**Groenewoudseweg 1**
**NL-5621 BA Eindhoven(NL)**

(84) **FR GB NL**

(72) Erfinder: **Harding, Geoffrey, Dr.**
**Poststrasse 18c**
**D-2083 Halstenbek(DE)**
Erfinder: **Kosanetzky, Josef-Marie, Dr.**
**Langenharmer Ring 120**
**D-2000 Norderstedt 1(DE)**
Erfinder: **Neitzel, Ulrich, Dr.**
**Kipps Weg 3**
**D-2000 Hamburg 65(DE)**

(74) Vertreter: **Hartmann, Heinrich, Dipl.-Ing. et al**
**Philips Patentverwaltung GmbH**
**Wendenstrasse 35 Postfach 10 51 49**
**D-2000 Hamburg 1(DE)**

(54) **Verfahren zur zweidimensionalen Compton-Profil-Abbildung.**

(57) Die Erfindung bezieht sich auf ein Verfahren zur Bestimmung des Compton-Profils eines in einem Untersuchungsbereich befindlichen Untersuchungsobjektes. Der Untersuchungsbereich wird dabei mit einem monochromatischen Primärstrahl durchsetzt, dessen Energie so gewählt ist, daß die Schwächung der Primärstrahlung im wesentlichen nur durch Compton-Streuung erfolgt. Die Streustrahlung wird energieaufgelöst gemessen und daraus sowie aus der Schwächung im Primärstrahl werden die Compton-Profile für die einzelnen Bildpunkte im Untersuchungsbereich bestimmt.

Fig. 3

Xerox Copy Centre

## "Verfahren zur zweidimensionalen Compton-Profil-Abbildung"

Die Erfindung betrifft ein Verfahren zur zweidimensionalen Compton-Profil-Abbildung, d.h. ein Verfahren, bei dem für einen Untersuchungsbereich das Comptonprofil als Funktion des Ortes bestimmt werden kann, sowie eine Anordnung zur Durchführung des Verfahrens.

Es ist bekannt, daß Compton-Streustrahlung eine größere Wellenlänge aufweist als die sie hervorrufende primäre Gamma-oder Röntgenstrahlung, und daß bei vorgegebener Wellenlänge der Primärstrahlung und bei vorgegebenem Austrittswinkel der Streustrahlung die Intensität der Streustrahlung bei einer definierten Wellenlänge ein Maximum hat. Jedoch ist beiderseits dieser Wellenlänge die Intensität der in den betreffenden Winkel gestreuten Strahlung nicht Null, so daß sich ein breiteres Intensitätsspektrum ergibt. Dieses Spektrum bzw. die Intensität der Compton-Streustrahlung als Funktion des Impulsübertrages (der in eindeutiger Weise durch die Wellenlänge und durch den Streuwinkel bestimmt wird) wird als Compton-Profil bezeichnet.

Neuere Untersuchungen haben gezeigt, daß das Compton-Profil eines biologischen Gewebes von dessen Beschaffenheit abhängt (Phys.Med.Biol., Vol. 28, p.l435, l983). Entsprechendes gilt auch für Halbleitermaterialien.

Aufgabe der vorliegenden Erfindung ist es daher, ein Verfahren zur zweidimensionalen Compton-Profilabbildung anzugeben, d.h. ein Verfahren, das es gestattet, die Compton-Profile in den einzelnen Bildpunkten einer Schicht zu bestimmen.

Diese Aufgabe wird erfindungsgemäß dadurch gelöst, daß ein Untersuchungsbereich mit einem monochromatischen Primärstrahl geringen Querschnitts durchstrahlt wird, dessen Energie so gewählt ist, daß die Schwächung des Primärstrahls im wesentlichen durch Comptonstreuung erfolgt, daß mit einem ersten Detektor die Intensität des Primärstrahls nach dem Durchsetzen des Untersuchungsbereichs und mit einem zweiten Detektor in Abhängigkeit von der Wellenlänge die Intensität der unter einem definierten Winkel in bezug auf den Primärstrahl austretenden Streustrahlung gemessen wird, daß die Messung für eine Vielzahl von parallelen Strahlenpfaden, die den Untersuchungsbereich in einer Vielzahl von Richtungen durchsetzen, wiederholt wird, daß aus den Meßwerten des ersten Detektors die Schwächung des Primärstrahls in deneinzelnen Bildpunkten rekonstruiert wird, daß die Compton-Streudichte in Abhängigkeit von der Quantenenergie an den Bildpunkten des Untersuchungsbereichs vorgegeben und daraus unter Berücksichtigung ihrer Schwächung für jeden Strahlenpfad die Intensität der Compton-Streustrahlung am Ort des zweiten Detektors berechnet wird, daß aus der Differenz der berechneten und der für den betreffenden Strahlenpfad gemessenen Intensität ein Koorekturwert zur Korrektur der vorgegebenen Compton-Streudichte an den einzelnen Bildpunkten gebildet wird.

Das erfindungsgemäße Abbildungsverfahren beruht darauf, daß zunächst für jeden Bildpunkt die auf den Streuwinkel bezogene Streudichte als Funktion der Energie vorgegeben wird. Davon ausgehend kann für jeden Bildpunkt der auf einem vom Primärstrahl bei der Messung durchsetzten Strahlenpfad liegt, die Intensität der Streustrahlung am Ort des zweiten Detektors als Funktion der Energie der Streustrahlung bestimmt werden - wenn die Intensität der Primärstrahlung bekannt ist und wenn die Schwächung bekannt ist, die die Strahlung auf ihrem Weg zu dem jeweiligen Bildpunkt (als Primärstrahlung) und von dort zum Detektor (als Streustrahlung) erfährt. Die Summe der von allen Bildpunkten auf einem Strahlenpfad gelieferten Streuintensitäten müßte der gemessenen Intensität entsprechen - wenn das vorgegebene Compton-Profil den tatsächlichen Verhältnissen entsprechen würde. In der Regel ist dies nicht der Fall; jedoch läßt sich aus der Differenz zwischen dem gerechneten und dem gemessenen Wert der Streuintensität ein Korrekturwert gewinnen, der auf die einzelnen Bildpunkte längs des Strahlenpfades verteilt wird. Nach dieser Korrektur stimmt das Compton-Profil, das auf diese Weise für die einzelnen Bildpunkte errechnet wird, schon besser mit seinem tatsächlichen Verlauf überein und diese Näherung wird durch weitere Iterationsschritte noch verbessert.

Zur Ermittlung des Compton-Profiles muß eine geeignete monochromatische Strahlenquelle verwendet werden, deren Strahlung eine solche Energie bzw. Wellenlänge hat, daß die Strahlung im wesentlichen nur durch Compton-Streuung geschwächt wird. Nur dann ist es nämlich möglich, aus dem Transmissions-Tomogramm, das mit Hilfe der Signale des ersten Detektors gebildet wird, die Schwächung der Streustrahlung, die eine niedrigere Energie hat bzw. eine größere Wellenlänge, rechnerisch zu bestimmen.

Eine Anordnung zur Durchführung dieses Verfahrens ist gekennzeichnet durch eine Strahlenquelle zur Erzeugung von monochromatischer Strahlung, eine Primärstrahlenblende zur Erzeugung eines den Untersuchungsbereich durchsetzenden Primärstrahls geringen Querschnitts, einen im Primärstrahl angeordneten ersten Detektor, einen die unter einem definierten Winkel austretende Streustrahlung erfassenden energieauflösenden zweiten Detektor, einen damit gekoppel ten Impulshöhenanalysator und mit einem mit dem Impulshöhenanalysator gekoppelten Rechner, der aus den Meßwerten der Detektoren iterativ das Comptonprofil in den Bildpunkten des Untersuchungsbereichs bestimm.

Eine bevorzugte Weiterbildung der Erfindung sieht vor, daß der Winkel, den die vom zweiten Detektor erfaßte Streustrahlung mit dem Primärstrahl einschließt, größer ist als 90°. Grundsätzlich kann die Erfindung zwar bei einem beliebigen Streuwinkel benutzt werden, doch ergeben sich besonders günstige Bedingungen, wenn die Rückwärts-Streustrahlung erfaßt wird, insbesondere bei einem Streuwinkel zwischen I50 und I60°.

Die Erfindung wird nachstehend anhand der Zeichnung näher erläutert. Es zeigen:

Fig. I ein Gerät zur Durchführung des erfindungsgemäßen Verfahrens,

Fig. 2 ein Flußdiagramm zur Bestimmung der Compton-Profile für die verschiedenen Bildpunkte,

Fig. 3 ein Flußdiagramm eines bei Fig. 2 benutzten Unterprogramms.

In Fig. I ist mit I eine Strahlenquelle dargestellt, von der ein Primärstrahl 2 geringen Querschnitts (pencil beam) mittels einer geeigneten Primärstrahlenblende 3 ausgeblendet wird. Der Primärstrahl 2 durchsetzt einen Untersuchungsbereich 4, in dem sich ein Untersuchungsobjekt 5, z.B. ein menschlicher Körper, auf einer Tischplatte befindet. Jenseits des Untersuchungsbereichs ist ein erster Detektor 6 angeordnet, der die Intensität des Primärstrahls 2 nach dem Durchsetzen des Untersuchungsbereichs 4 mißt.

Die Strahlenquelle I, die Primärstrahlenblende 3 und der Detektor 6 sind mechanisch auf nicht näher dargestellte Weise miteinander gekoppelt. Sie können durch geeignete Antriebe in der Zeichenebene horizontal verschoben und um das Zentrum des Untersuchungsbereichs 4 gedreht werden. Dadurch kann der Untersuchungsbereich 4 mit dem Primärstrahl 2 auf einer Vielzahl paralleler Strahlenpfade und aus einer Vielzahl unterschiedlicher Richtungen abgetastet werden.

Das Ausgangssignal des ersten Detektors 6 wird über einen Verstärker 7 einem Analog-Digital-Wandler 8 zugeführt, so daß der damit gekoppelte Rechner 9 für jede Richtung eines jeden Strahlenpfades ein digitales Datenwort erhält. Daraus kann der Rechner ein sogenanntes Transmissions-Tomogramm erstellen, das die Verteilung der Schwächung-die im wesentlichen durch Compton-Streuung bestimmt ist-in der vom Primärstrahl 2 abgetasteten Ebene des Untersuchungsbereichs 4 bestimmen kann.

Insoweit als bisher beschrieben, entspricht die Anordnung den bekannten Computer-Tomographen der ersten Generation. Allerdings ist die Strahlenquelle eine monochromatische Strahlenquelle, die Gammaquanten mit einer Energie von einigen I00 keV emittiert, beispielsweise das Radioisotop Gold I98, das Gammaquanten mit einer Energie von 4I2 keV liefert.

Zusätzlich umfaßt die Anordnung einen zweiten Detektor I0, der mechanisch mit den Elementen I, 3 und 6 fest gekoppelt ist. Dieser Detektor, der die unter einem Winkel von etwa I55° (d.h. nach rückwärts) gestreute Streustrahlung erfaßt, besitzt ein Energieauflösungsvermögen, d.h. er liefert Impulse, deren Amplitude proportional zur Energie des einfallenden Gammaquants ist. Der Detektor kann ein Halbleiter-Detektor sein und beispielsweise aus Ge oder CdTe o.dgl. bestehen.

Der Detektor I0 ist über einen Verstärker II und einen Analog-Digital-Wandler I2 mit den Adreß-Eingängen eines Speichers I3 verbunden. Der Analog-Digital-Wandler ist genügend schnell und erzeugt bei jeder Detektion eines Röntgenquants durch den vorgeschalteten Detektor I0 ein Signal, das der Amplitude des Ausgangssignals des Detektors entspricht. Jedesmal, wenn auf diese Weise ein Spannungspuls in ein digitales Datenwort umgesetzt wird, wird die diesem Datenwort entsprechende Adresse in dem Speicher I2 aufgerufen. Mit dem gleichen Takt, mit dem die Analog-Digital-Wandlung erfolgt, wird ein Addierer I4 aktiviert, der zu dem Inhalt des aufgerufenen Speicherplatzes eine Eins addiert und das Ergebnis in den gleichen Speicher zurückschreibt. Für jeden Strahlenpfad, auf dem der Primärstrahl den Untersuchungsbereich durchsetzt, werden auf diese Weise die Röntgenquanten gezählt, deren Quantenenergie jeweils etwa den gleichen Wert hat. Somit wirken der Digital-Wandler I2, der Speicher I3 und der Addierer I4 als Impulshöhenanalysator, der einen Satz von Meßwerten $M(EI)$, $M(E2)$...$M(Et)$ liefert, die die Intensität der Streustrahlung für verschiedene Energiebereiche EI, E2...Et darstellen.

In dem Rechner 9 wird daraus sowie aus dem mit Hilfe der Ausgangssignale des Detektors 6 errechneten Transmissions-Computertomogramm die auf den Streuwinkel (der durch die Lage des zweiten Detektors I0 in bezug auf den Untersuchungsbereich 4 vorgegeben ist) bezogene Streudichte für jeden Bildpunkt und für jede Energie bzw. Impulsübertrag berechnet. Am Schluß der Berechnung ist für jeden

3

Impulsübertrag in einer Speicheranordnung l5 ein Bild der Streueigenschaften des Untersuchungsbereichs 4 gespeichert. Die Bilder können mit Hilfe eines Monitors l6 dargestellt werden. Es ist jedoch auch möglich, für jeden Bildpunkt - oder über mehrere Bildpunkte gemittelt - die Streueigenschaften als Funktion des Impulsübertrages, d.h. das Compton-Profil, darzustellen.

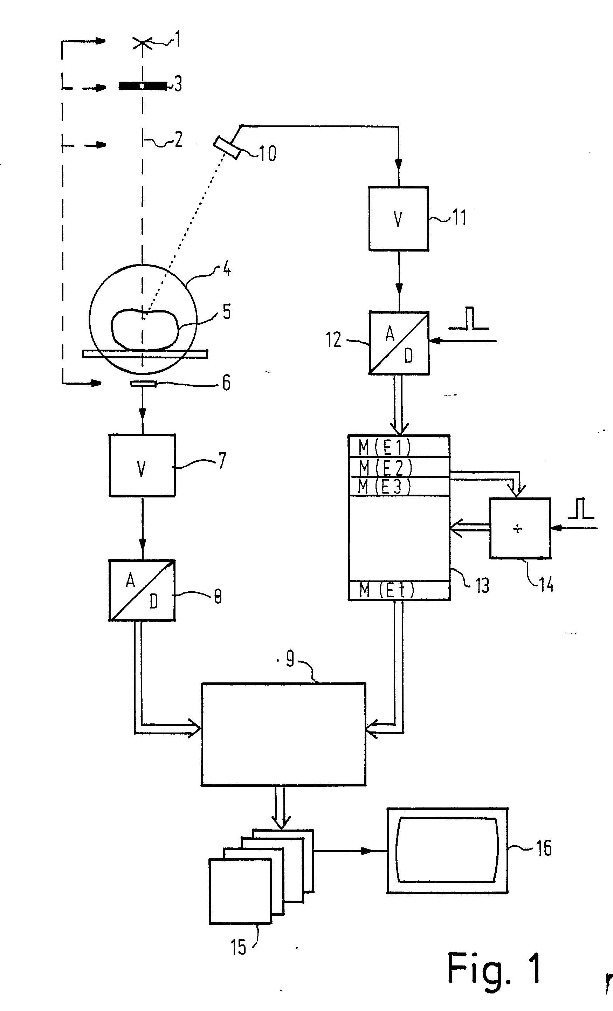

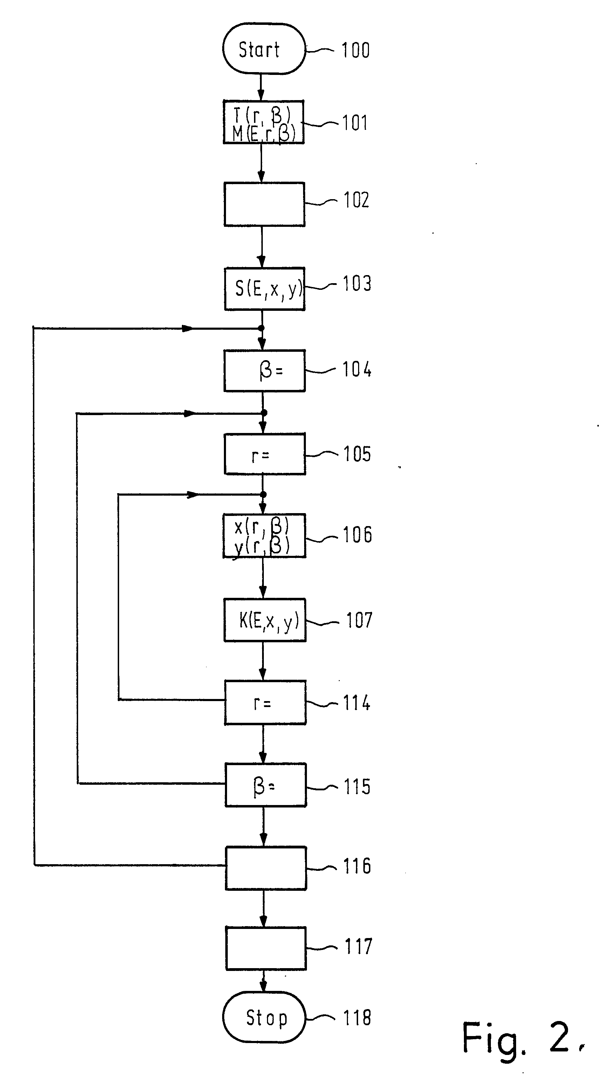

Nachstehend wird nun das Verfahren zur Bestimmung der Compton-Profile anhand des in Fig. 2 dargestellten Flußdiagramms näher erläutert. Nach dem Start (Block l00) erfolgt die Erfassung der Meßwerte der Detektoren 6 und l0. Der Detektor 6 liefert einen Satz von Meßwerten $\bar{T}(r,\beta)$, die ein Maß für die Transmission auf dem Strahlenpfad in Abhängigkeit von dem Abstand r dieses Strahlenpfades vom Zentrum des Untersuchungsbereiches 4 und von dem Winkel $\beta$ abhängen, unter dem der Strahlenpfad den Untersuchungsbereich durchsetzt. Der Detektor l0 liefert einen Satz von Meßwerten M(E,r,$\beta$), der ebenfalls von r und abhängt, jedoch auch von der Energie E der Streustrahlung.

Aus den Meßwerten T(r,$\beta$) wird die Schwächungsverteilung in der vom Primärstrahl durchsetzten Ebene des Untersuchungsbereiches 4 bestimmt (Block l02). Die hierfür erforderlichen Rekonstruktionsverfahren sind dem Fachmann der Computertomographie hinreichend bekannt, so daß sich ein näheres Eingehen hierauf erübrigt.

Im nächsten Schritt des Programms (Block l03) wird der Streukoeffizient S(E,x,y) vorgegeben. Der Streukoeffizient stellt das Produkt aus der Streudichte und des auf den vom Detektor l0 erfaßten Raumwinkel sowie auf den jeweiligen Energiebereich (El,E2...Et) bezogenen differentiellen Streuquerschnitts dar. Der Streukoeffizient eines Bildpunktes wäre unmittelbar dem vom Detektor l0 und dem Impulshöhenanalysator l2 bis l4 gelieferten Meßwert für den betreffenden Energiebereich proportional - wenn im Untersuchungsbereich außerhalb des Bildpunktes keine Streuung erfolgen würde. Es ist möglich, die Streukoeffizienten S(E,x,y) aus dem Computertomogramm abzuleiten, indem die Energieabhängigkeit für alle Bildpunkte zunächst gleich angenommen und mit dem Schwächungswert für den betreffenden Bildpunkt multipliziert wird. Es ist jedoch auch möglich, den Streukoeffizienten S(E,x,y) für jeden Bildpunkt und für jeden Energiebereich als Konstante einzusetzen. In diesem Fall muß die Vorgabe nicht innerhalb des Rekonstruktionsverfahren erfolgen; vielmehr können diese Werte schon vor Beginn des Verfahrens in einem Speicher abgelegt sein.

In den beiden nächsten Schritten wird die Lage eines Strahlenpfades vorgegeben, in dem der Winkel $\beta$ vorgegeben wird, unter dem der Strahlenpfad den Untersuchungsbereich durchsetzt (Block l04), und indem der Abstand r des Strahlenpfades vom Zentrum des Untersuchungsbereiches vorgegeben wird (Block l05).

Anschließend werden in einem kartesischen xy-Koordinatensystem, dessen Ursprung mit dem Zentraum des Untersuchungsbereichs zusammenfällt und dessen y-Achse von dem Strahlenpfad unter dem Winkel geschnitten wird, die Koordinaten x(r,$\beta$) und y(r,$\beta$) der Bildpunkte bestimmt, die im Untersuchungsbereich auf dem Strahlenpfad liegen (Block l06). Diese Bestimmung erfolgt nach der Gleichung

$$x \cos\beta + y \sin\beta = r \qquad (1)$$

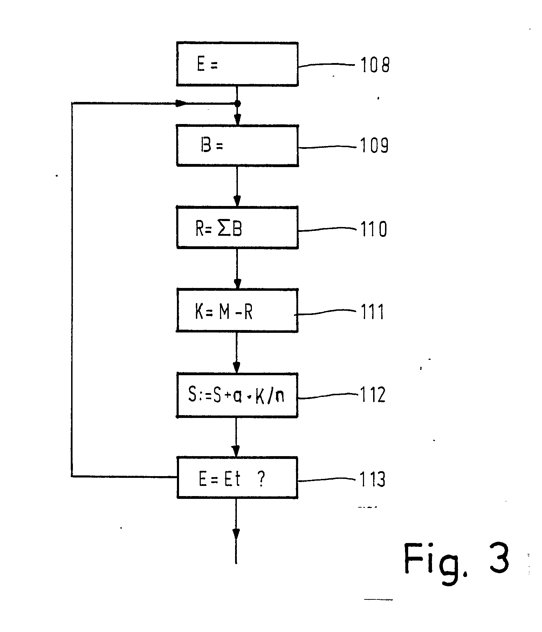

Anschließend wird (Block l07) für die Bildpunkte, die auf dem durch r und $\beta$ definierten Strahlenpfad liegen, und für jeden der Energiebereiche ein Korrekturwert gebildet, der zu dem gespeicherten Wert des Streukoeffizienten für den gleichen Energiebereich und den gleichen Bildpunkt hinzuaddiert wird. Dieser Korrekturwert ist so gewählt, daß die Abweichung zwischen dem vorgegebenen Wert des Streukoeffizienten und dem tatsächlichen Wert verringert wird. Wie dies geschieht, wird im folgenden anhand von Fig. 3 erläutert.

Für einen bestimmten Energiebereich (E = El - Block l08) wird der Beitrag B errechnet, den ein Bildpunkt zu dem von der Anordnung l0...l4 erzeugten Signal liefert. Dieser Beitrag berechnet sich nach der Gleichung

$$B = c^* S\, {}^*Fp^*\, Fs \qquad (2)$$

Dabei ist S der Streukoeffizient, Fp die Schwächung, die der Primärstrahl auf dem Strahlenpfad bis zu dem jeweiligen Bildpunkt erfährt (d.h. der Bruchteil der Primärstrahlung, der den Bildpunkt erreicht), Fs die Schwächung, die die von dem Bildpunkt ausgehende Streustrahlung bis zum Detektor l0 erfährt (d.h. der Anteil der im Bildpunkt erzeugten und in Richtung des Detektors l0 verlaufenden Streustrahlung, der den Detektor l0 erreicht) und c eine Konstante, die von dem Untersuchungsbereich 4 unabhängig ist und die nur von den Eigenschaften des übrigen Gerätes (z.B. der Intensität der Strahlenquelle l, der Empfindlichkeit des Detektors l0 usw.) abhängt. Der Faktor Fp kann dem zuvor (Block l02) berechneten Computertomogramm unmittelbar entnommen werden da daraus die Schwächung der Bildpunkte bekannt ist, die auf dem Strahlenpfad zwischen dem betreffenden Bildpunkt und der Strahlenquelle liegen.

4

Der Faktor Fs kann dem Tomogramm nicht unmittelbar entnommen werden weil die Quantenenergie der gestreuten Strahlung wesentlich niedriger ist als die der Primärstrahlung; beträgt beispielsweise die Quantenenergie im Primärstrahl 412 keV, dann ergibt sich für einen Streuwinkel von 155° das Maximum der Compton-Streuung bei einer Energie von etwa 162,4 keV. Streustrahlung mit einer soviel niedrigeren Energie wird wesentlich stärker geschwächt als die Energie der Primärstrahlung. Da jedoch die Energie der Primärstrahlung so gewählt ist, daß die Schwächung der Strahlung im wesentlichen durch Compton-Streuung erfolgt und da das Verhältnis der Schwächungen durch Compton-Streuung bei verschiedenen Energien von den Streueigenschaften des Körpers unabhängig ist, ergibt sich die Schwächung der Sekundärstrahlung aus der Schwächung im Computertomogramm, wenn man die Schwächungswerte in den zwischen dem betreffenden Bildpunkt und dem zweiten Detektor 10 liegenden Bildpunkten mit einem konstanten Faktor multipliziert. Der Faktor entspricht dem totalen Streuquerschnitt bei der Energie der Streustrahlung dividiert durch den totalen Streuquerschnitt bei der Energie des Primärstrahls. Die Berechnung der totalen Streuquerschnitte für verschiedene Energien ist aus der Literatur bekannt (z.B. Hubbel "Photon Cross Sections, Attenuation Coefficients ...", herausgegeben im August 1969 von National Bureau of Standards). Für die angegebenen Enerien der Streustrahlung beträgt dieser Faktor etwa 1,4.

Auf entsprechende Weise werden die Beiträge aller anderen Bildpunkte auf dem vom Primärstrahl durchsetzten Strahlenpfad berechnet und summiert (Block 110). Der Summenwert R dieser Beiträge müßte dem vom Detektor bzw. dem Impulshöhenanalysator ermittelten Meßwert M für die gleiche Energie und für den gleichen Strahlenpfad entsprechen wenn die vorgegebene Verteilung des Streukoeffizienten (Block 103) mit der im Untersuchungsbereich tatsächlich vorhandenen übereinstimmen würde. Da dies in der Regel nicht der Fall ist, wird ein Korrekturwert gebildet (Block 111), der der Differenz zwischen dem Meßwert M und dem errechnete Wert R entspricht jeweils für den gleichen Strahlenpfad r,ß und für die gleiche Energie. Der so bestimmte Korrekturwert K wird durch die Zahl der Bildpunkte auf dem Strahlenpfad dividiert und mit einem Gewichtungsfaktor a, der kleiner ist als Eins, zu dem für den betreffenden Bildpunkt xy und die betreffende Energie vorgegebenen Wert S hinzuaddiert (Block 112). Der so gebildete Wert kommt dem tatsächlichen Wert schon näher als der zunächst vorgegebene.

Die Schritte 109 bis 112 werden für die anderen Energiebereiche E2, E3 usw. wiederholt, bis - Block 113 - die Korrektur auch für den letzten Energiebereich E = Et durchgeführt worden ist.

Danach wird ein neuer Strahlenpfad bestimmt, indem der Wert r, d.h. der Abstand des Strahlenpfades vom Mittelpunkt des Untersuchungsbereiches 4 variiert wird (Block 114), so daß ein zum bisherigen Strahlenpfad benachbarter und paralleler Strahlenpfad vorgegeben wird. Für diesen Strahlenpfad werden die Schritte 106...113 wiederholt, wonach der nächste Strahlenpfad vorgegeben wird usw., bis die Korrektur für alle den Untersuchungsbereich unter dem Winkel durchsetzenden Strahlenpfade erfolgt ist.

Danach wird eine andere Richtung der Strahlenpfade vorgegeben (Schritt 115) und die Schritte 105 bis 114 werden wiederholt, bis alle Winkelstellungen, unter denen der Primärstrahl bei der Messung den Untersuchungsbereich durchsetzt hat, verarbeitet sind.

Auf diese Iteration können dann noch (Block 116) weitere Iterationen folgen, wobei die Schritte 104 bis 115 mehrmals durchlaufen werden. Dabei kann eine feste Zahl von Iterationen vorgesehen sein. Es ist jedoch auch möglich, die Iteration dann abzubrechen, wenn die Korrekturen (Block 111) stets einen bestimmten Schwellenwert unterschreiten. Danach liegt eine korrigierte Verteilung S(E,x,y) vor, die der tatsächlichen Verteilung in guter Näherung entspricht.

Zwischen der Quantenenergie E der Streustrahlung und dem Impulsübertrag Q besteht bekanntlich die Beziehung

$$Q = 1/c * \sqrt{E^2_p + E^2 - 2E\, E_p \cos a} \qquad (3),$$

wobei c die Lichtgeschwindigkeit, $E_p$ die Quantenenergie des Primärstrahls und a der Streuwinkel ist. Infolgedessen lassen sich die für jeden Bildpunkt nach dem vorstehend beschriebenen Verfahren iterativ ermittelten Werte S(E) umrechnen in Werte S(Q), die gegebenenfalls noch so normiert werden können, daß das Integral $\int S(Q)dQ$ gleich dem Streukoeffizienten des Transmissionsbildes für den betreffenden Bildpunkt ist und die den Verlauf des Compton-Profils für den betreffenden Bildpunkt darstellen. Danach ist die Rekonstruktion beendet (Block 118).

Da zwischen dem Impulsübertrag und der Energie der gestreuten Quanten bei vorgegebenem Streuwinkel eine eindeutige Beziehung besteht, können bereits die im Schritt l0l für die einzelnen Strahlenpfade als Funktion der Energie ermittelten Meßwerte auch als Funktion des Impulsübertrages angegeben werden. Im Block l03 werden dann die Streukoeffizienten für die einzelnen Bildpunkte als Funktion des Impulsübertrages vorgegeben, wonach die geschilderte Umrechnung des Streustrahlenspektrums in ein Compton-Profil im Block ll7 entfallen kann.

**Ansprüche**

l. Verfahren zur zweidimensionalen Compton-Profil-Abbildung,
dadurch gekennzeichnet, daß ein Untersuchungsbereich (4) mit einem monochromatischen Primärstrahl (2) geringen Querschnitts durchstrahlt wird, dessen Energie so gewählt ist, daß die Schwächung des Primärstrahls im wesentlichen durch Comptonstreuung erfolgt, daß mit einem ersten Detektor (6) die Intensität des Primärstrahls nach dem Durchsetzen des Untersuchungsbereichs und mit einem zweiten Detektor (l0) in Abhängigkeit von der Wellenlänge die Intensität der unter einem definierten Winkel in bezug auf den Primärstrahl austretenden Streustrahlung gemessen wird, daß die Messung für eine Vielzahl von parallelen Strahlenpfaden, die den Untersuchungsbereich in einer Vielzahl von Richtungen durchsetzen, wiederholt wird, daß aus den Meßwerten des ersten Detektors die Schwächung des Primärstrahls (T(r,ß)) in den einzelnen Bildpunkten rekonstruiert wird, daß die Compton-Streudichte in Abhängigkeit von der Quantenenergie an den Bildpunkten (S(E,x,y)) des Untersuchungsbereichs vorgegeben und daraus unter Berücksichtigung ihrer Schwächung (Fp, Fs) für jeden Strahlenpfad die Intensität der Compton-Streustrahlung am Ort des zweiten Detektors (l0) berechnet wird, daß aus der Differenz der berechneten (R) und der für den betreffenden Strahlenpfad gemessenen (M) Intensität ein Korrekturwert zur Korrektur der vorgegebenen Compton-Streudichte an den einzelnen Bildpunkten gebildet wird.

2. Anordnung zur Durchführung des Verfahrens nach Anspruch l,
gekennzeichnet durch eine Strahlenquelle (l) zur Erzeugung von monochromatischer Strahlung, eine Primärstrahlenblende (3) zur Erzeugung eines den Untersuchungsbereich durchsetzenden Primärstrahls geringen Querschnitts, einen im Primärstrahl angeordneten ersten Detektor (6), einen die unter einem definierten Winkel (a) austretende Streustrahlung erfassenden energieauflösenden zweiten Detektor (l0), einen damit gekoppelten Impulshöhenanalysator (l2...l4) und mit einem mit dem Impulshöhenanalysator gekoppelten Rechner (9), der aus den Meßwerten der Detektoren iterativ das Compton-Profil in den Bildpunkten des Untersuchungsbereichs bestimmt.

3. Anordnung nach Anspruch 2,
dadurch gekennzeichnet, daß als Strahlenquelle (l) ein radioaktives Isotop, vorzugsweise Gold l98, dient.

4. Anordnung nach Anspruch 2,
dadurch gekennzeichnet, daß der Winkel (a), den die vom zweiten Detektor (l0) erfaßte Streustrahlung mit dem Primärstrahl einschließt, größer ist als 90°.

## Fig. 1

1-Ⅲ-PHD 86-123

Fig. 2.

Fig. 3